# EUROPÄISCHE PATENTSCHRIFT

(11) **EP 2 483 673 B1**
(45) Veröffentlichungstag und Bekanntmachung des Hinweises auf die Patenterteilung: **31.10.2018**
(21) Anmeldenummer: 10760335.9
(22) Anmeldetag: 29.09.2010
(51) Int. Cl.: G01N 27/416, C12M 1/34, G01N 33/487

(54) **ANORDNUNG UND VERFAHREN UNTER VERWENDUNG VON MIKROSENSOREN ZUM MESSEN VON ZELL-VITALITÄTEN**
APPARATUS AND METHOD USING MICROSENSORS FOR MEASURING CELL ACTIVITY
PROCÉDÉ ET APPAREIL UTILISANT DES MICROCAPTEURS POUR MESURER L'ACTIVITÉ DE CELLULES

(30) Priorität: 30.09.2009 DE 102009043527
(43) Veröffentlichungstag der Anmeldung: 08.08.2012
(73) Patentinhaber: Boehringer Ingelheim Vetmedica GmbH, 55216 Ingelheim am Rhein (DE)
(72) Erfinder: GUMBRECHT, Walter, 91074 Herzogenaurach (DE); SCHIENLE, Meinrad, 85521 Ottobrunn (DE); FREY, Alexander, 81737 München (DE); PAULICKA, Peter, 91341 Röttenbach (DE); SICKERT, Daniel, 90403 Nürnberg (DE); STANZEL, Manfred, 92334 Berching (DE)
(74) Vertreter: Von Rohr Patentanwälte Partnerschaft mbB
(86) Internationale Anmeldenummer: PCT/EP2010/064437
(87) Internationale Veröffentlichungsnummer: WO 2011/039242

(56) Entgegenhaltungen:
- WO-A1-2009/073121
- DE-A1-102005 027 245
- U. FREY, U. EGERT, F. HEER, S. HAFIZOVIC, A. HIERLEMANN: "Microelectronic system for high-resolution mapping of extracellular electric fields applied top brain slices", BIOSENSORS AND BIOELECTRONICS, Bd. 24, 7. Dezember 2008 (2008-12-07), Seiten 2191-2198, XP002614091,
- SOLE S ET AL: "New materials for electrochemical sensing III. Beads", TRAC, TRENDS IN ANALYTICAL CHEMISTRY, ELSEVIER, AMSTERDAM, NL, Bd. 20, Nr. 2, 1. Februar 2001 (2001-02-01), Seiten 102-110, XP004249003, ISSN: 0165-9936, DOI: DOI:10.1016/S0165-9936(00)00059-5

## Beschreibung

Die vorliegende Erfindung umfasst eine Anordnung und ein Verfahren zum Messen von Zell-Vitalitäten mit einem Sensor-Array. Das Sensor-Array ist auf einer Oberfläche eines Halbleiter-Chips ausgebildet, wobei der Halbleiter-Chip integrierte Schaltungen umfasst und jedem Sensor des Sensor-Arrays eine integrierte Schaltung zugeordnet ist, zur Verarbeitung der Messsignale des jeweiligen Sensors. Die integrierten Schaltungen sind in dem Halbleiter-Chip räumlich jeweils unterhalb des zugeordneten Sensors ausgebildet. Ein pH-Wert und/oder ein pO₂-Wert kann in der Umgebung einer lebenden Zelle gemessen werden.

Die DE 10 2005 027 245 A1 beschreibt eine Schaltkreisanordnung für eine Mehrzahl von Sensorelementen auf einem Substrat. Der Betriebsschaltkreis der Anordnung ist örtlich vom Sensor-Bereich getrennt und weist einen Auswerteschaltkreis auf, der eine Vielzahl elektronischer Komponenten, beispielsweise Verstärkereinheiten, umfasst, um Sensorereignisse zu detektieren.

Die Publikation "Microelectronic system for high-resolution mapping of axtracellular electric fields applied top brain slices" von Frey et. al., erschienen in Biosensors and Bioelectronics im Dezember 2008, offenbart einen CMOS-basierten Chip mit einem Mikroelektroden-Array, bei dem jede Elektrode mit einem Auslesekanal verbunden werden kann, der sich außerhalb des Arrays auf dem Chip befindet. Der Auslesekanal nimmt die Messsignale der Elektroden auf und umfasst elektrische Verstärker.

Die WO 2009/073121 A1 beschreibt einen Elektroden-Array, bei dem jede Elektrode mit einer Schaltung verbunden ist, die neben einem Transistor auch einen Verstärker und einen Kondensator umfasst.

In dem Artikel "New materials for electrochemical sensing III. Beads" von Solé et. al., erschienen in Trends in Analytical Chemistry im Februar 2001, wird die Verwendung magnetischer Partikel in Biosensoren beschrieben, wobei elektrochemische Messsignale detektiert werden.

Darüber hinaus sind in der Mikrobiologie eine Vielzahl von Methoden zur Untersuchung von pathogenen Keimen auf der Basis von Zellkultur- und Antibiotika-Resistenztests bekannt. Ein Vorteil ist der "phänotypische" Ansatz, bei welchem die Wirkung, wie z.B. das Wachstum bzw. die Hemmung des Zellwachstums, untersucht wird. Über die Wirkung auf Zellkulturen kann ein direkter Bezug zur Wirkung auf Menschen oder Tieren gewonnen werden. Zellkulturen werden dabei in einer Nährlösung über Tage hinweg z.B. in Petrischalen eingelegt und beobachtet. Das Wachstum bzw. die Schädigung der Zellkulturen wird über lange Zeiträume hinweg gemessen und ausgewertet. Die langen Zeiträume, welche für die Beobachtung notwendig sind, machen das Verfahren sehr aufwendig und langwierig.

Zur Messung des Wachstums bzw. der Schädigung der Zellkulturen können Sensorsysteme verwendet werden. Lebende Zellen werden z.B. auf Sensoren aufgewachsen, um danach die Vitalität der Zellen z.B. durch Messung von Impedanz, Sauerstoff (pO₂-Wert) oder pH-Wert zu überwachen. Als Sensoren können Interdigitalelektroden-Arrays, Sauerstoff-Sensoren oder pH-Wert-Sensoren verwendet werden. Masse für die Vitalität der
Zellen sind unter anderem deren Adhäsion auf Oberflächen, deren Atmung oder deren Stoffwechsel. Das Aufwachsen der Zellen auf den Sensoren ist jedoch zeitaufwändig und führt zu einer eingeschränkten Lagerfähigkeit der Sensorsysteme. Aufgewachsene Zellen auf den Sensoren können auf der Oberfläche wandern und/oder absterben.

Für eine Messung der Vitalität von Zellen über einen Sauerstoff-Wert oder pH-Wert ist es notwendig, dass sich lebende Zellen in unmittelbarer Nähe zu Sensoren befinden. Nur so kann sichergestellt werden, dass Konzentrationsänderungen von Ausgangssubstanzen oder Reaktionsprodukten des Zellstoffwechsels durch die Sensoren registriert werden. Ein direktes Aufwachsen der Zellen auf die Elektroden muss dabei verhindert werden, da für eine zuverlässige Messung z.B. durch elektrochemische Sensoren ein Flüssigkeitsfilm zwischen Zellwand und Sensoroberfläche vorliegen muss.

Zellen weisen eine Größenordnung im Bereich von Mikrometern auf. Übliche elektrochemische Sensoren bestehen aus Metalloberflächen, welche z.B. in Form von kammförmig ineinandergreifenden Interdigital-Elektroden aufgebaut sind, mit einem kreisrunden Gesamtumfang eines Sensors. Der Durchmesser eines solchen Sensors liegt in der Regel im Bereich von mehreren Millimetern. Um ein Übersprechen der Sensoren zu verhindern, weisen die Sensoren einen Abstand voneinander auf, welcher ebenfalls im Bereich von Millimetern liegt. Häufig sind zwischen Sensoren wandförmige Stege ausgebildet, um eine bessere Trennung der Sensoren voneinander und Zuordnung von Signalen zu Bereichen über den Sensoren zu erhalten sowie ein Übersprechen effektiv zu unterdrücken.

Einzelne lebende Zellen führen in ihrer Umgebung durch ihren Stoffwechsel nur zu geringen Konzentrationsänderungen von chemischen bzw. biochemischen Stoffen. Diese geringen Konzentrationsänderungen sind nur messbar, wenn Sensoren eine hohe Sensitivität für die Substanz aufweisen und die Zellen nah genug an den Sensoren angeordnet sind. Sensoren mit einem Gesamtdurchmesser im Bereich von Millimetern sind schwerlich in der Lage eine Sensitivität zu erreichen, welche ausreicht Stoffwechselprodukte einzelner Zellen zu messen. Nebenreaktionen auf großen Elektrodenflächen führen zu schlechten Signal-Rauschverhältnissen. Ein großer Abstand zwischen den Sensoren kann dazu führen, dass Zellen zwischen den Sensoren angeordnet sind und keine Signale der Zellen gemessen werden können.

Der Abstand zwischen den Mittelpunkten zweier Sensoren bzw. die mögliche höchste Packungsdichte von Sensoren eines Sensor-Arrays ist mitbestimmt durch die Kontaktierung und Anordnung von signalverarbeitenden Schaltungen. So können bei Verwendung von Halbleiterträgern integrierte Schaltungen direkt unter den Sensoren angeordnet sein. Die integrierten Schaltungen können jeweils für einen Sensor unter dem Sensor ausgebildet sein, z.B. bei Verwendung von CMOS-Technologie im Siliziumträgermaterial. Die Größe einer Schaltung bestimmt dann die mögliche größte Packungsdichte der jeweils zugeordneten, darüber befindlichen Sensoren des Sensor-Arrays. Eine übliche integrierte Schaltung zur Messung von elektrochemischen Signalen eines Sensors weist einen Platzverbrauch bzw. eine Fläche im Träger des Sensor-Arrays auf, welcher im Millimeterbereich liegt. Insbesondere die Realisierung von Operationsverstärkern in integrierten Schaltungen führt zu einem hohen Platzverbrauch.

Aufgabe der vorliegenden Erfindung ist es deshalb, eine Anordnung und ein die Anordnung verwendendes Verfahren zum Messen von Zell-Vitalitäten anzugeben, wobei jedem Sensor eines Sensor-Arrays zur Verarbeitung der Messsignale des Sensors eine integrierte Schaltung zugeordnet ist. Ferner ist es Aufgabe eine Anordnung anzugeben, welche sicherstellt, dass im Wesentlichen alle auf dem Sensor-Array befindlichen Zellen registriert bzw. gemessen werden können. Eine weitere Aufgabe ist es, eine besonders platzsparende integrierte Schaltung anzugeben, welche eine hohe Packungsdichte der Sensoren im Sensor-Array erlaubt. Damit soll eine schnelle und einfache sowie zuverlässige Messung von Parametern ermöglicht werden, welche typisch für die Zell-Vitalität sind.

Die angegebene Aufgabe wird bezüglich der Anordnung zum Messen von Zell-Vitalitäten durch die Merkmale des Anspruchs 1 und bezüglich des Verfahrens unter Verwendung der Anordnung zur Bestimmung von Zell-Vitalitäten durch die Merkmale des Anspruchs 12 gelöst.

Vorteilhafte Ausgestaltungen der erfindungsgemäßen Anordnung und des Verfahrens zur Bestimmung von Zell-Vitalitäten gehen aus den jeweils zugeordneten abhängigen Unteransprüchen hervor. Dabei können die Merkmale des Hauptanspruchs mit Merkmalen der Unteransprüche und Merkmale der Unteransprüche untereinander kombiniert werden.

Die erfindungsgemäße Anordnung zum Messen von Zell-Vitalitäten umfasst ein Sensor-Array, welches auf einer Oberfläche eines Halbleiter-Chips ausgebildet ist. Dabei umfasst der Halbleiter-Chip integrierte Schaltungen und jedem Sensor des Sensor-Arrays ist zur Verarbeitung der Messsignale des jeweiligen Sensors eine integrierte Schaltung zugeordnet. Die integrierten Schaltungen in dem Halbleiter-Chip sind räumlich jeweils unterhalb des zugeordneten Sensors ausgebildet. Benachbarte Sensoren des Sensor-Arrays weisen einen Abstand der Mittelpunkte der benachbarten Sensoren im Bereich von Mikrometern auf.

Die geringe Größe und der geringe Abstand der Sensoren voneinander ermöglicht eine Messung von geringen Konzentrationsänderungen chemischer bzw. biochemischer Stoffe in der Umgebung eines Sensors. Störende Nebenreaktionen führen durch die geringe Fläche eines Sensors zu keinem großen Signal. Räumlich stark beschränkte geringe Konzentrationsänderungen können so zuverlässig registriert und gemessen werden.

Der Abstand der Mittelpunkte von benachbarten Sensoren kann in der Größenordnung von lebenden Zellen liegen, insbesondere
im Bereich von 1 bis 100 Mikrometern. Bevorzugt liegt der Abstand der Mittelpunkte von benachbarten Sensoren im Bereich von 1 bis 10 Mikrometern, was der üblichen Größe einer Zelle entspricht. Dadurch ist sichergestellt, dass alle auf dem Sensor-Array befindlichen Zellen vermessen werden können. Zellen im Zwischenraum zwischen zwei Sensoren liegen immer dicht genug an einem Sensor, um die Stoffwechselprodukte oder Änderungen der Konzentration der Ausgangssubstanzen der Stoffwechselprodukte zuverlässig und mit einem guten Signal-Rausch-Verhältnis zu messen.

Die Sensoren können elektrochemische Sensoren sein, insbesondere amperometrische oder coulometrische elektrochemische Sensoren. Elektrochemische Sensoren können auf engstem Raum zuverlässig Konzentrationsänderungen von chemischen oder biochemischen Substanzen messen, auch in optisch eingetrübten Lösungen. Die magnetischen Partikel führen zu keiner Verschlechterung des Signal-Rausch-Verhältnisses, wie es z.B. bei optischen Messungen der Fall wäre. Eine Anordnung von magnetischen Einrichtungen kann im Gegensatz zu optischen Messungen keinen störenden Einfluss auf die Anordnung der elektrochemischen Messeinrichtung ausüben, da elektrochemische Messanordnungen sehr platzsparend nur elektrische Signale wandeln, transportieren und verarbeiten müssen. Eine reine elektrische Messung ist kostengünstiger, einfacher und platzsparender als z.B. optische Messungen. Die Sensoren können jeweils wenigstens eine Interdigital-Elektrode als Arbeitselektrode umfassen. Interdigital-Elektroden ermöglichen sehr empfindliche elektrochemische Messung. Alternativ können die Arbeitselektroden auch als durchgehende, z.B. kreisförmige Fläche ausgebildet sein. Referenz- und Gegenelektroden können am Rand des Sensor-Arrays angeordnet sein oder in Zwischenräumen zwischen Arbeitselektroden. Die Anordnung der Referenz- und Gegenelektroden am Rand des Sensor-Arrays ermöglicht einen geringeren Abstand von benachbarten Arbeitselektroden des Sensor-Arrays voneinander und ermöglicht somit eine zuverlässigere Messung aller auf dem Array befindlichen Zellen bzw. deren Zell-Vitalität.

Die amperometrischen oder coulometrischen Messungen führen zu einem Verbrauch an Stoffen, welche ebenfalls von Zellen umgesetzt werden. Eine kleine aktive Sensorfläche, welche im Mikrometerbereich liegt, sorgt dafür, dass wenig Stoffumsatz an der Elektrode stattfindet. Bei gleichzeitigem Stoffumsatz durch die Zelle ist bei einem großen Wert des Verhältnisses Stoffumsatz Zelle zu Stoffumsatz Elektrode der Stoffumsatz der Zelle besser zu messen und der Sensor ist empfindlicher für Änderungen in der Vitalität der Zellen.

Bei amperometrischen oder coulometrischen Messungen werden bei chemischen Reaktionen Ladungsträger an den Sensoren umgesetzt, welche dann als Messsignal dienen. Bei kleinen zu messenden Konzentrationen an Stoffen, welche durch die Zellen umgesetzt werden, werden an den Sensoren nur geringe Mengen an Ladungsträgern umgesetzt und entstehen somit nur kleine Messesignale. Um diese verarbeiten zu können, müssen Leitungswege kurz gehalten werden, da elektrische Verluste in den Leitungen zu einem Signalsverlust führen. In der Regel werden deshalb die Signale der Sensoren nahe einem Sensor direkt verstärkt oder verarbeitet. Dazu werden in dem Halbleitermaterial direkt unter einem Sensor integrierte Schaltungen angeordnet. Elektrische Bauelemente von integrierten Schaltungen, wie z.B. Operationsverstärker oder Kondensatoren führen zu einem hohen Flächenverbrauch im Halbleitermaterial. Um kleine Sensoren zu ermöglichen, welche dicht gepackt auf einer Oberfläche in Array-Form angeordnet sind, müssen integrierte Schaltungen unterhalb der Sensoren angeordnet werden, welche jeweils in der Größenordnung eines Sensors liegen.

Die integrierte Schaltung eines Sensors umfasst zwei Schalt-Transistoren zum Schalten des Sensors, insbesondere der Arbeitselektrode des jeweiligen Sensors. Dabei ermöglichen die Transistoren ein Schalten der an der Arbeitselektrode anliegenden Spannung zwischen zwei Spannungswerten, einer ersten und einer zweiten Spannung. Integrierte Schaltungen ohne Bauelemente wie z.B. Operationsverstärker oder
Kondensatoren, hauptsächlich oder ausschließlich umfassend Transistoren, führen zu einem geringen Flächenverbrauch einer integrierten Schaltung im Halbleitermaterial. Dadurch wird eine dichte Packung der über den integrierten Schaltungen angeordneten kleinen Sensoren möglich.

Die integrierte Schaltung eines Sensors umfasst einen Transistor als Spannungsfolger und einen Auswahltransistor, zum gezielten elektrischen Auswählen des jeweiligen Sensors nach Spalte und Zeile des Sensor-Arrays. Die Transistoren, insbesondere ausgeführt in CMOS-Technoiogie, benötigen wenig Platz im Halbleitermaterial. Jeder Sensor des Sensor-Arrays kann einzeln angesteuert und ausgelesen werden. Die elektrochemische Messung selbst wird durch das Schalten der Schalt-Transistoren von der ersten auf die zweite Spannung erreicht. Über den Transistor als Spannungsfolger und den Auswahltransistor wird ein Sensor jeweils ausgelesen.

Die integrierte Gesamtschaltung kann zur Unterdrücken von elektrischen Rauschsignalen und zur Unterdrückung von elektrischer Drift eines Sensors für jeden Sensor eine korrelierte Doppel-Sampling-Stufe (CDS) umfassen, welche unterhalb des zugeordneten Sensors oder in einem Bereich des Halbleiter- Chips außerhalb des Bereichs des Sensor-Arrays angeordnet ist. Die Anordnung der Doppel-Sampling-Stufen (CDS) in einem Bereich des Halbleiter-Chips außerhalb des Bereichs des Sensor-Arrays führt zu einer besonders hohen Integrationsdichte der integrierten Schaltungen unterhalb der Sensoren.

Die erfindungsgemäße Anordnung kann weiterhin Mittel zum Ein- stellen einer definierten Temperatur über dem Sensor-Array umfasst. Insbesondere eine Temperatur von 37°C ist für die Vitalität der Zellen günstig. Das Einstellen, und über die gesamte Messezeit Konstanthalten dieser Temperatur, ermöglicht einen Vergleich von unterschiedlichen Messungen beziehungsweise ergibt vergleichbare Bedingungen über die gesamte Messezeit hinweg.

Die Anordnung kann Mittel zum fixieren von lebenden Zellen über Sensoren des Sensor-Arrays umfassen. Frei bewegliche Zellen können auf einer Oberfläche wandern, was eine elektrochemische Messung stören kann. Gerade in Hinblick eines Monitorrings von Messwerten über längere Zeiträume hinweg, muss ein Wandern von Zellen auf dem Sensor-Array verhindert werden.

Dazu kann als Mittel zum fixieren von lebenden Zellen eine Filtermembran dienen, welche in fluidischem Kontakt mit den Sensoren des Sensor-Arrays angeordnet ist. Durch Austausch der Filtermembran können abgestorbene Zellen nach einer Messung entfernt werden und die Anordnung zum Messen von Zell-Vitalitäten für eine weitere Messung regeneriert bzw. vorbereitet werden durch Auflegen einer neuen Filtermembran mit frischen, lebenden Zellen auf das Sensor-Array.

Alternativ kann die Anordnung wenigstens eine Magnetfeld erzeugende Einrichtung umfassen, welche ausgebildet ist, ein Magnetfeld über den Sensoren des Sensor-Arrays zu erzeugen. Lebende Zellen können an magnetische Partikel gebunden sein und durch das Magnetfeld über dem Sensor-Array fixiert werden. Besonders günstig für eine Messung ist dabei, wenn dies in Form einer im Wesentlichen gleichmäßig dicken Schicht erfolgt, welche aus magnetischen Partikeln mit in der Matrix von magnetischen Partikeln eingebetteten Zellen aufgebaut ist.

Das Magnetfeld kann zwischen einem Ein- und einem Aus-Zustand schaltbar sein. Dadurch können tote oder geschädigte Zellen im Zustand des ausgeschalteten Magnetfeldes entfernt werden, z.B. durch einen Flüssigkeitsstrom.

Der Halbleiter-Chip kann von einer Durchflusszelle umfasst sein und die Sensoren des Sensor-Arrays können in fluidischem Kontakt mit einem Durchflusskanal der Durchflusszelle angeordnet sein. Dies ergibt einen besonders einfachen Messaufbau für die Anordnung zum Messen von Zell-Vitalitäten.

Ein erfindungsgemäßes Verfahren zum Messen von Zell-Vitalitäten unter Verwendung der zuvor beschriebenen Anordnung umfasst, dass von wenigstens einem Sensor des Sensor-Arrays der pH-Wert und/oder der pO₂-Wert in der Umgebung einer lebenden Zelle gemessen wird. Alternativ können auch Proteine gemessen werden. Diese Stoffe stellen Ausgangsstoffe oder Reaktionsprodukte des Zellstoffwechsels dar. Die Messung der geringen Änderungen der Stoffkonzentrationen, verursacht durch eine Zelle, werden durch die geringe Größe und die dichte Packung der Sensoren im Sensor-Array erst ermöglicht. Durch den Abstand der Mittelpunkte zweier benachbarter Sensoren im Bereich von Mikrometern, wird ein Verbrauch von z.B. Sauerstoff durch einen Sensor so weit reduziert, dass der geringe Verbrauch von z.B. Sauerstoff durch eine Zelle in der Nachbarschaft zu dem Sensor gemessen werden kann.

Dabei ist es günstig, wenn über dem Sensor-Array eine für die Zell-Vitalität optimale Temperatur eingestellt wird, insbesondere im Bereich von 37°C, und/oder Substanzen den Zellen zugeführt werden, welche die Zell-Vitalität begünstigen oder aufrechterhalten, insbesondere Nährlösung und/oder Sauerstoff, und/oder Substanzen den Zellen zugeführt werden, welche die Zell-Vitalität verschlechtern, insbesondere Antibiotika.

Die mit dem Verfahren zum Messen von Zell-Vitalitäten unter Verwendung der Anordnung verbundenen Vorteile sind analog den Vorteilen, welche zuvor im Bezug auf die Anordnung zum Messen von Zell-Vitalitäten beschrieben wurden.

Bevorzugte Ausführungsformen der Erfindung mit vorteilhaften Weiterbildungen gemäß den Merkmalen der abhängigen Ansprüche werden nachfolgend anhand der Figuren näher erläutert, ohne jedoch darauf beschränkt zu sein.

Es wird in den Figuren dargestellt:
- Fig. 1: eine schematische Schnittdarstellung durch einen Halbleiter-Chip mit Sensoren eines Sensor-Arrays und darauf befindlicher Schicht aus magnetischen Partikeln mit eingebetteten Zellen, und
- Fig. 2: eine Schematische Darstellung einer integrierten Schaltung zum elektrochemischen Messen mit einem Sensor nach dem Stand der Technik, und
- Fig. 3: eine Schematische Darstellung einer integrierten Schaltung zum elektrochemischen Messen mit einem Sensor-Array unter Verwendung einer CDS-Stufe, und
- Fig. 4: eine Schematische Darstellung einer platzsparenden, erfindungsgemäßen integrierten Schaltung zum elektrochemischen Messen mit Sensoren eines Sensor-Arrays aufgebaut ausschließlich aus Transistoren.

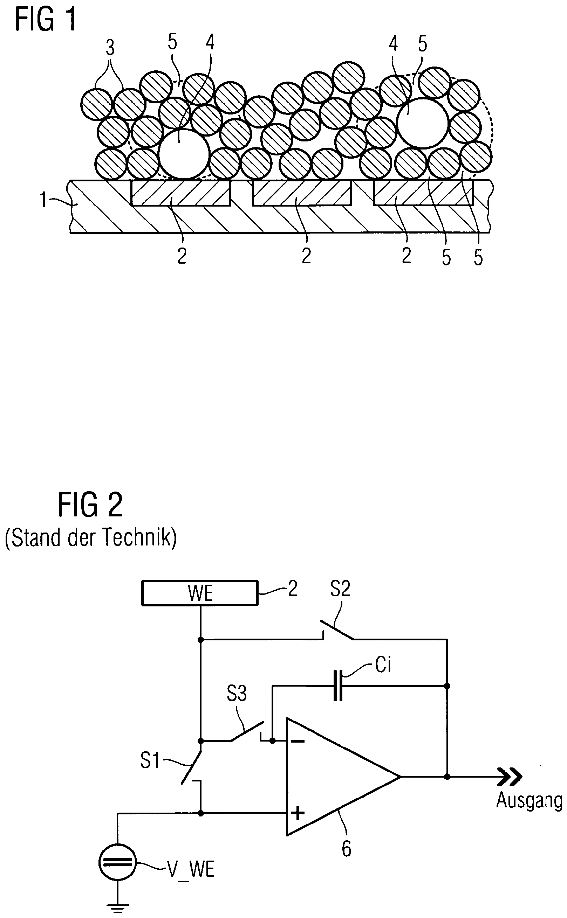

In Fig. 1 ist eine Anordnung aus in einer Matrix von magnetischen Partikeln 3 angeordneten Zellen 4 auf Sensoren 2 eines Sensor-Arrays in einer Schnittdarstellung gezeigt. Das Sensor-Array ist auf einem Halbleiter-Chip 1 ausgebildet, welcher integrierte Schaltungen zum elektrochemischen Messen mit Hilfe der Sensoren 2 umfasst. Die magnetischen Partikel 3 sind magnetisch durch nicht gezeigte Magnetfeld erzeugende Mittel auf der Oberfläche des Chips 1 in Form einer im Wesentlichen gleichmäßig dicken Schicht fixiert. Die Zellen 4 können an die magnetischen Partikel 3 z.B. durch Antikörper gebunden sein oder auf die Partikel aufgewachsen sein. Die magnetischen Partikel 3 weisen eine Größe im Bereich von Mikrometern oder Nanometern auf, und die Zellen weisen eine Größe im Bereich von einigen Mikrometern auf. Bei lebenden Zellen können in der direkten Umgebung 5 der Zellen 4 Stoffwechselprodukte gemessen werden sowie der Verbrauch von für den Stoffwechsel notwendigen Stoffen wie z.B. Sauerstoff.

Die Sensoren 2 zum elektrochemischen Messen der Stoffwechselprodukte und/oder Ausgangsstoffe der Stoffwechselprodukte sind aus einem Metall, z.B. Gold aufgebaut. Eine dünne Goldschicht ist auf den Chip 1 als Trägermaterial aufgebracht, wobei z.B. Zwischenschichten als Haftvermittler zwischen Goldschicht und Trägermaterial dienen können. Die Sensoren 2 aus z.B. der Goldschicht sind in kreisrunder Form ausgebildet mit einem Mittelpunkt, welcher dem Kreismittelpunkt entspricht. Die kreisrunden Sensoren 2 sind in Spalten und Zeilen als Array auf der Chipoberfläche angeordnet, wobei die Mittelpunkte der Sensoren 2 jeweils an den Schnittpunkten der Zeilen und Spalten des Arrays angeordnet sind. Das Trägermaterial ist aus einem Halbleitermaterial wie z.B. Silizium, wobei in dem Material integrierte Schaltungen zum Ansteuern und Verarbeiten der an den Sensoren 2 gemessenen elektrochemischen Signale ausgebildet sind. Die Schaltung kann z.B. durch CMOS-Technik im Halbleitermaterial realisiert werden.

In Fig. 2 ist eine Schaltung zum Ansteuern und elektrochemischen Messen mit einem Sensor dargestellt, wie sie aus dem Stand der Technik bekannt ist. Der Einfachheit halber ist die Referenz und Gegenelektrode nicht dargestellt. Die Arbeitselektrode WE bildet den Sensor 2 und kann in kreisrunder Form oder als Interdigitalelektrode ausgeführt sein. In einem Ausführungsbeispiel kann mit Hilfe des Sensor-Arrays, aufgebaut aus Sensoren 2 in Zeilen und Spalten angeordnet auf der ChipOberfläche, Sauerstoff in Form des pO₂-Wertes in einer Flüssigkeit gemessen werden. Der pO₂-Wert ist ein Maß für die Vitalität von lebenden Zellen 4, welche sich nahe einem Sensor 2 befinden. Eine lebende Zelle 4 verbraucht beim Stoffwechsel Sauerstoff und die Abnahme der Sauerstoffkonzentration in ihrer nähe kann gemessen werden.

Dazu wird die Array-Oberfläche mit luftgesättigter Nährlösung versorgt. Der Flüssigkeitsstrom wird für kurze Zeit angehalten und über die Sensoren 2 wird die Sauerstoffkonzentration in Form des pO₂-Wertes gemessen. Die pO₂-Werte bleiben konstant, es sei denn ein Sauerstoff-Verbraucher in Form einer lebenden Zelle 4 lässt den pO₂-Wert lokal abfallen. Dies wird durch den nächstgelegenen Sensor 2 registriert, unter der Voraussetzung eines im Vergleich zum Sauerstoffverbrauch der Zelle 4 geringen oder nicht vorhandenen Sauerstoffverbrauchs durch den Sensor 2 bei der Messung, und unter der Voraussetzung eines geringen Abstands zwischen Zelle 4 und Sensor 2. Über die Lage des Sensors 2 im Sensor-Array kann auf die Lage der lebenden Zelle 4 geschlossen werden. Die lebenden Zellen 4 auf dem Sensor-Array werden so räumlich identifiziert.

Anschließend wird das Sensor-Array mit Antibiotika versetzter Nährlösung überströmt und der Flüssigkeitsstrom wird gestoppt. Sind die zuvor räumlich identifizierten Zellen 4 jeweils sensitiv auf das entsprechende Antibiotikum, so wird an dem jeweilig nächstgelegenen Sensor 2 kein Abfall des pO₂-Wertes gemessen. An Sensorpositionen, an denen lebende Zellen 4 angeordnet sind, welche nicht sensitiv sind bezüglich des entsprechenden Antibiotikums, wird weiterhin eine Abnahme des pO₂-Wertes entsprechend dem Zellstoffwechsel gemessen.

Um eine Verfälschung der Messwerte des pO₂-Wertes durch Sauerstoffverbrauch der Sensoren 2 selbst bei amperometrischen oder coulometrischen Messungen zu verhindern oder zu reduzieren, kann ein Pulsverfahren verwendet werden. Der SauerstoffPartialdruck wird typischerweise in der Elektrochemie mit einem amperometrischen Verfahren gemessen, d.h. ein Faraday' scher Strom wird als Maß für die Sauerstoffkonzentration verwendet, welcher bei coulometrischer Messung über die Zeit aufintegriert wird. Sauerstoff wird dabei an der Elektrode bzw. am Sensor 2 umgesetzt und somit verbraucht. Dies führt zu einer Veränderung der Sauerstoff-Konzentration und somit zu einer Verfälschung des Messwertes des Sauerstoffverbrauchs durch die Zelle 4. Durch Verwendung von kurzen Messzeiten, d.h. einem Pulsverfahren, kann dieser Effekt reduziert werden. Die Sensoren 2 werden zur Messung nur kurzzeitig kathodisch polarisiert.

Bei zu kurzen Pulsen führt die Polarisierung jedoch zu einem Stromfluss durch Umladung der Flüssigkeits- bzw. Elektrolyt-Doppelschicht-Kapazität über dem Sensor 2. Der Vorgang ist schnell abgeschlossen, so dass nur ein kurzer Stromfluss stattfindet. Dabei ist der Stromfluss abhängig von der Fläche des Sensors 2. Je kleiner die elektrochemisch aktive Fläche des Sensors 2 ist, desto geringer fällt der störende Effekt durch die Umladung der Doppelschicht aus, und desto schneller ist der Stromfluss verursacht durch Umladung der Doppelschicht abgeschlossen. Nachdem der kapazitive Stromfluss durch Umladung der Doppelschicht abgeklungen ist, kann der sich einstellende Stromfluss als Maß für den SauerstoffPartialdruck verwendet werden. Anschließend wird der Sensor 2 bzw. die Elektrode auf Ruhepotential geschaltet, um weitern Sauerstoffumsatz durch die Elektrode zu vermeiden.

Alternativ zur kathodischen Polarisierung des Sensors 2 kann durch kurzzeitige anodische Polarisierung des Sensors 2 bzw. der Elektrode ein Recycling der Reaktionsprodukte wie z.B. H₂O₂ zu Sauerstoff erfolgen. Anstelle eines Ruhepotentials kann auch eine geeignete anodische Polarisierung des Sensors 2 erfolgen, um zumindest einen Teil des umgesetzten Sauerstoffes wiederzugewinnen.

Durch das Einbetten der Zellen 4 in einem "Gitter" bzw. einer Matrix von magnetischen Partikeln bzw. Magnet-Beads 3, wie es in Fig. 1 dargestellt ist, werden definierte Flüssigkeitsräume zwischen den Partikeln 3 bzw. den Zellen 4 und den Sensoren 2 geschaffen. Bei einer Schichtdicke der Schicht von magnetischen Partikel 3 mit eingebetteten Zellen 4, welche im Bereich 5 der Änderung der Messgröße liegt, welche durch den Stoffwechsel einer Zelle 4 erzeugt wird, ist ein empfindlicher elektrochemischer Nachweis der Vitalität der Zelle sichergestellt. Durch Einengung der Flüssigkeitsräume in der Umgebung der Zellen 4 durch die magnetischen Partikel 3, wird die Empfindlichkeit der Messung gegenüber frei vorliegenden Zellen 4 in Flüssigkeit ohne magnetische Partikel 3 erhöht.

Alternativ zu dem pO₂-Wert können auch Stoffwechselprodukte der Zelle wie Säuren über den pH-Wert gemessen werden. Die pH-Wert-Änderung ist analog dem pO₂-Wert umso größer, je kleiner das die Zellen 4 umgebende Flüssigkeitsvolumen ist.

Das amperometrische Signal eines Sensors 2 kann mit Hilfe der in Fig. 2 gezeigten Schaltung nach dem Stand der Technik in ein Spannungs-Signal umgesetzt werden. Die Integratorschaltung integriert den Strom in einem definierten Zeitintervall nach dem gepulsten Anlegen der Polarisierung an einem Sensor 2 und wandelt das Ergebnis in eine Spannung um.

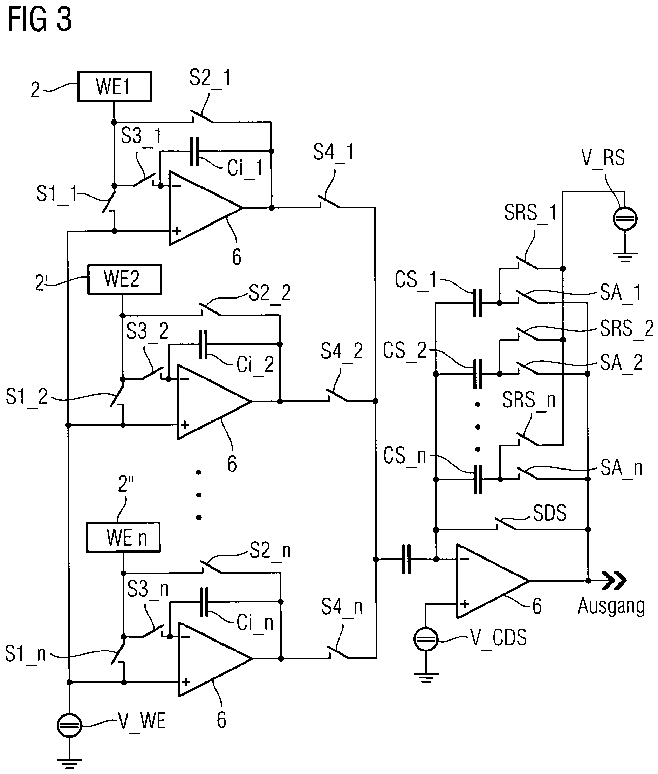

Zur Unterdrückung von Offsetsignalen kann eine Doppel-Sampling-Stufe oder eine Korrelierte-Doppel-Sampling-Stufe (CDS), wie sie in Fig. 3 dargestellt ist, verwendet werden. Dabei wird der Nullpunktseffekt in enger zeitlicher Korrelation zum Messsignal erfasst. Dies kann direkt vor oder nach dem ermitteln des Messsignals erfolgen. Hierdurch werden alle Driften und Rauschanteile, die vor der CDS-Stufe entstehen, wirksam unterdrückt. Bei einem Sensor-Array benötigt nicht jeder Sensor 2 notwendigerweise eine eigene CDS-Stufe. Vielmehr können die CDS-Stufen in einem Randbereich des Chips 1 außerhalb der Sensor-Array-Fläche angeordnet werden. Jede Stufe ist dann z.B. einer Spalte des Sensor-Arrays zugeordnet.

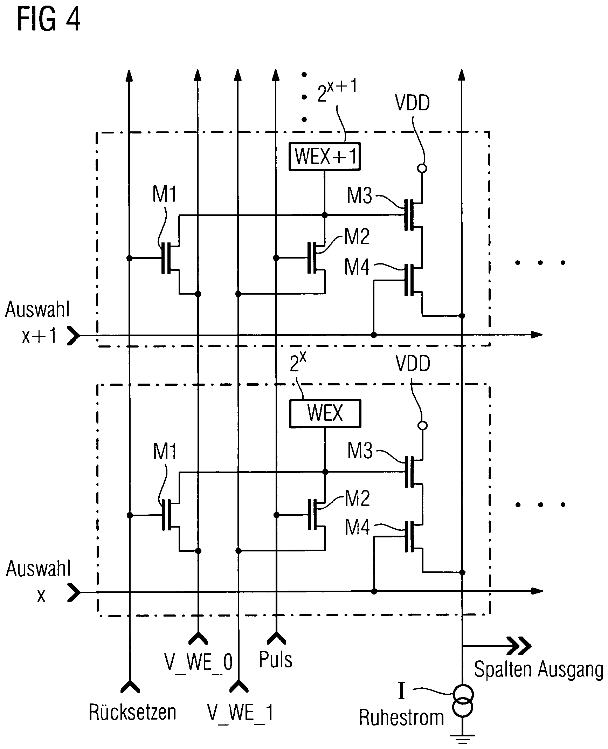

Ein erfindungsgemäßer Aufbau einer integrierten Schaltung zum Messen mit einem Sensor-Array ist in Fig. 4 dargestellt. Dabei sind die CDS-Stufen am Rand des Halbleiter-Chips 1 angeordnet und der Einfachheit halber in Fig. 4 nicht dargestellt. Es kann unter Umständen auch auf die CDS-Stufen verzichtet werden.

In der in Fig. 4 gezeigten integrierten Schaltung wird die Spannung an den Sensoren 2 bzw. Arbeitselektroden WE_{X} nicht durch einen Verstärker 6 geregelt, wie es in Fig. 2 der Fall ist, sondern durch Schalter werden die Sensoren 2 bzw. Arbeitselektroden WE_{X} direkt mit den gewünschten Spannungen V_WE_0 und V_WE_1 verbunden. Dadurch werden Sensorschaltungen mit minimalem Bedarf an Bauelementen wie z.B. Transistoren und an Chipfläche bzw. Chipoberfläche möglich. Die über der jeweiligen integrierten Schaltung angeordneten Sensoren 2 können im Vergleich zum Stand der Technik mit einer höheren Integrationsdichte angeordnet werden, und somit können kleinere Sensorflächen und/oder geringere Sensorabstände gewählt werden, um das Sensor-Array aufzubauen. Dies hat die zuvor beschriebenen Vorteile, d.h. es können keine Zellen 4 zwischen Sensoren 2 in der magnetischen Partikel-Matrix angeordnet sein, welche nicht von Sensoren 2 in ihrer Vitalität gemessen werden können bzw. deren Stoffumsatz nicht gemessen werden kann, unter der Voraussetzung einer kleinen Schichtdicke. Die kleineren elektrochemisch aktiven Sensor-Oberflächen ergeben eine sensitive Messung z.B. von Sauerstoffumsatz oder anderen Größen beeinflusst vom Zellstoffwechsel, ohne bzw. mit nur geringem störendem Stoffumsatz an den Sensoren 2 selbst.

Die in Fig. 4 dargestellte integrierte Schaltung kommt mit nur 4 Transistoren M1 bis M4 je Sensor 2 bzw. Elektrode WE_{X} aus. Die Transistoren M1 und M2 dienen als Schalt-Transistoren bzw. Schalter, über welche die Arbeitselektrode WE_{X} bzw. der Sensor 2^{X} jeweils wahlweise mit der Spannung V_WE_0 oder V_WE_1 versorgt wird. X steht dabei für die Zeile des anzusteuernden Sensors 2^{X}. Zu Beginn einer Messung mit einem Sensor 2^{X} wird die Elektrode WE_{X} bzw. die elektrochemisch aktive Fläche des Sensors 2^{X} mit dem Potential V_WE_0 verbunden, durch Schließen des Schalters M1. Das Potential V_WE_0 ist so gewählt, dass keine elektrochemische Reaktion der für die Zell-Vitalität typischen Messgröße an der Arbeitselektrode WE_{X} erfolgt. Dann wird der Schalter M1 geöffnet und durch Schließen des Schalters M2 wird die Elektrode WE_{X} auf ein Potential V_WE_1 gebracht. Anschließend wird der Schalter M2 wieder geöffnet. Durch die elektrochemische Reaktion entlädt sich die Elektrode WE_{X} mit einer Zeitkonstante, welche durch die Doppelschichtkapazität und den elektrochemischen Strom bestimmt ist. Nach einer definierten Zeit wird die Spannung der Elektrode WE_{X} über die Transistoren M3 und M4 ausgelesen. M3 fungiert dabei als Spannungsfolger und M4 ist der Auswahltransistor, über welchen der Sensor 2 mit der Position X im Sensor-Array ausgewählt wird und mit dem Ausgang Column Out verbunden wird. Die ausgelesene Spannung steht dann am Ausgang Column Out zur weitern Verarbeitung zur Verfügung. Zur Nullpunktsunterdrückung kann am Ausgang Column Out auch die CDS-Stufe der Fig. 3 angeschlossen sein.

Mit der Schaltung aus Fig. 4 können Sensor-Arrays realisiert werden, welche einen Abstand der Mittelpunkte benachbarter Sensoren 2^{X} und 2^{X+1} bzw. Array-Raster von bis zu 10µm aufweisen. Elektrische Bauelemente, wie z.B. Operationsverstärker 6 oder Kondensatoren Ci müssen in der integrierten Schaltung direkt unterhalb eines jeweiligen Sensors 2 nicht realisiert werden, wodurch eine hohe Integrationsdichte der Schaltung direkt unterhalb eines Sensors 2 erreicht wird. Auf dem Chip 1 können integrierte Schaltungen z.B. am Randbereich enthalten sein, welche eine weitere Signalverarbeitung ermöglichen. Es können auf dem Chip 1 integrierte Schaltungen wie z.B. Potentiostaten oder integrierte Schaltungen zur Strommessung außerhalb des Bereichs des Sensor-Arrays angeordnet sein.

Die Anordnung und das Verfahren zum Messen von Zell-Vitalitäten können in Umwelt- und Pharmauntersuchungen eingesetzt werden, z.B. um toxische Stoffe zu identifizieren. So können z.B. Schadstoffe in Wasser oder Luft untersucht werden oder die Wirkung von Medikamenten auf z.B. Tumorzellen untersucht werden. Dabei kann eine definierte Anzahl von lebenden Zellen dem Sensor-Array zugeführt werden und nach einer Messung können geschädigte oder tote Zellen wieder entfernt werden. Dann können durch Zufuhr frischer lebender Zellen weitere Messungen erfolgen.

Die Anordnung und das Verfahren zum Messen von Zell-Vitalitäten können aber auch in Gesundheitsuntersuchungen eingesetzt werden. Dabei kann eine Identifikation einer unbestimmten Zahl bestimmter Zellen, wie z.B. Staphylococcus Aureus Bakterien in Analogie zu einem mikrobiologischen Nachweis auf Nährböden erfolgen. Diese Identifikation kann aufgrund der empfindlicheren Messtechnik viel schneller erfolgen, als die zeitaufwendigen Nachweise durch Züchtung der Zellkulturen bis sie optisch im Licht-Mikroskop sichtbar sind. Durch die Verwendung von Sensor-Arrays mit einer hohen Sensor-Packungsdichte können, ähnlich wie in Petri-Schalen, lokale Keime über die aus ihnen hervorgehenden Kolonien, rein über die Zellen der Keime mit hoher räumlicher Auflösung identifiziert werden. Es können an den Keimen Antibiotika-Resistenztest z.B. zum Erkennen von MRSA durchgeführt werden, durch Zuführen von steigenden Konzentrationen bestimmter Antibiotika und Messung der Zell-Vitalität.

## Patentansprüche

1. Anordnung zum Messen von Zell-Vitalitäten, mit einem Sensor-Array, welches auf einer Oberfläche eines Halbleiter-Chips (1) ausgebildet ist, wobei der Halbleiter-Chip (1) integrierte Schaltungen umfasst und jedem Sensor (2) des Sensor-Arrays eine integrierte Schaltung zugeordnet ist zur Verarbeitung der Messsignale des jeweiligen Sensors (2), wobei die integrierten Schaltungen in dem Halbleiter-Chip (1) räumlich jeweils unterhalb des zugeordneten Sensors (2) ausgebildet sind und wobei benachbarte Sensoren (2) des Sensor-Arrays einen Abstand der Mittelpunkte der benachbarten Sensoren (2) im Bereich von Mikrometern aufweisen,
**dadurch gekennzeichnet,**
**dass** die integrierte Schaltung eines Sensors (2) zwei Schalt-Transistoren (M1 , M2) umfasst zum Schalten des Sensors (2) zwischen zwei Spannungswerten, einer ersten und einer zweiten Spannung und
**dass** die integrierte Schaltung eines Sensors (2) einen Transistor als Spannungsfolger (M3) und einen Auswahltransistor (M4) zum gezielten elektrischen Auswählen des jeweiligen Sensors (2) nach Spalte und Zeile des Sensor-Arrays umfasst.

2. Anordnung nach Anspruch 1, **dadurch gekennzeichnet, dass** der Abstand der Mittelpunkte von benachbarten Sensoren (2) in der Größenordnung von lebenden Zellen (4), insbesondere im Bereich von 1 bis 100 Mikrometern, bevorzugt im Bereich von 1 bis 10 Mikrometern liegt.

3. Anordnung nach Anspruch 1 oder 2, **dadurch gekennzeichnet, dass** die Sensoren (2) elektrochemische Sensoren sind, insbesondere amperometrische oder coulometrische elektrochemische Sensoren, und/oder die Sensoren (2) jeweils wenigstens eine Interdigital-Elektrode als Arbeitselektrode umfassen.

4. Anordnung nach einem der vorhergehenden Ansprüche, **dadurch gekennzeichnet, dass** die integrierte Schaltung zur Unterdrücken von elektrischen Rauschsignalen und zur Unterdrückung von elektrischer Drift eines Sensors (2) für jeden Sensor (2) eine korrelierte Doppel-Sampling-Stufe (CDS) umfasst, welche unterhalb des zugeordneten Sensors (2) oder in einem Bereich des Halbleiter-Chips (1) außerhalb des Bereichs des Sensor-Arrays angeordnet ist.

5. Anordnung nach einem der vorhergehenden Ansprüche, **dadurch gekennzeichnet, dass** die Anordnung Mittel zum Einstellen einer definierten Temperatur über dem Sensor-Array umfasst.

6. Anordnung nach einem der vorhergehenden Ansprüche, **dadurch gekennzeichnet, dass** die Anordnung Mittel zum fixieren von lebenden Zellen (4) über Sensoren (2) des Sensor-Arrays umfasst.

7. Anordnung nach Anspruch 6, **dadurch gekennzeichnet, dass** als Mittel zum Fixieren von lebenden Zellen (4) eine Filtermembran in fluidischem Kontakt mit den Sensoren (2) des Sensor-Arrays angeordnet ist.

8. Anordnung nach Anspruch 6, **dadurch gekennzeichnet, dass** die Anordnung wenigstens eine Magnetfeld erzeugende Einrichtung umfasst, welche ausgebildet ist ein Magnetfeld über den Sensoren (2) des Sensor-Arrays zu erzeugen, und wobei lebende Zellen (4) an magnetische Partikel (3) gebunden sind.

9. Anordnung nach Anspruch 8, **dadurch gekennzeichnet, dass** die lebenden Zellen (4) durch das Magnetfeld über dem Sensor-Array fixiert sind, in Form einer im Wesentlichen gleichmäßig dicken Schicht aus magnetischen Partikeln (3) mit in der Matrix von magnetischen Partikeln (3) eingebetteten Zellen (4).

10. Anordnung nach Anspruch 9, **dadurch gekennzeichnet, dass** das Magnetfeld zwischen einem Ein- und einem Aus-Zustand schaltbar ist, insbesondere zum Entfernen von toten oder geschädigten Zellen (4) im Zustand des ausgeschalteten Magnetfeldes.

11. Anordnung nach einem der vorhergehenden Ansprüche, da- durch gekennzeichnet, dass der Halbleiter-Chip (1) von einer Durchflusszelle umfasst ist und die Sensoren (2) des Sensor-Arrays in fluidischen Kontakt mit einem Durchflusskanal der Durchflusszelle angeordnet sind.

12. Verfahren zum Messen von Zell-Vitalitäten mit einer Anordnung nach einem der vorhergehenden Ansprüche, **dadurch gekennzeichnet, dass** von wenigstens einem Sensor (2) des Sensor-Arrays der pH-Wert und/oder der pO₂-Wert in der Umgebung einer lebenden Zelle (4) gemessen wird.

13. Verfahren nach Anspruch 12, **dadurch gekennzeichnet, dass** über dem Sensor-Array eine für die Zell-Vitalität optimale Temperatur eingestellt wird, insbesondere im Bereich von 37°C, und/oder Substanzen den Zellen (4) zugeführt werden, welche die Zell-Vitalität begünstigen, insbesondere Nährlösung und/oder Sauerstoff, und/oder Substanzen den Zellen (4) zugeführt werden, welche die Zell-Vitalität verschlechtern, insbesondere Antibiotika.

## Claims

1. Arrangement for measuring cell vitalities, with a sensor array which is formed on a surface of a semiconductor chip (1), wherein the semiconductor chip (1) comprises integrated circuits and an integrated circuit is assigned to each sensor (2) of the sensor array for processing the measurement signals of the respective sensor (2), wherein the integrated circuits in the semiconductor chip (1) are each spatially formed under the assigned sensor (2), and wherein adjacent sensors (2) of the sensor array have a distance between midpoints of the adjacent sensors (2) in the micrometer range,
**characterized in**
**that** the integrated circuit of a sensor (2) comprises two switching transistors (M1, M2) for switching the sensor (2) between two voltage values, a first and a second voltage, and
**that** the integrated circuit of a sensor (2) comprises a transistor as voltage follower (M3) and a selection transistor (M4) for the specific electrical selection of the respective sensor (2) by column and row of the sensor array.

2. Arrangement according to claim 1, **characterized in that** the distance between midpoints of the adjacent sensors (2) lies in the order of size of living cells (4), in particular in the range from 1 to 100 micrometers, preferably in the range from 1 to 10 micrometers.

3. Arrangement according to claim 1 or 2, **characterized in that** the sensors (2) are electrochemical sensors, in particular amperometric or coulometric electrochemical sensors, and/or the sensors (2) each comprise at least one interdigital electrode as a working electrode.

4. Arrangement according to any one of the previous claims, **characterized in that** the integrated circuit for suppressing electrical noise signals and for suppressing electrical drift of a sensor (2) comprises for each sensor (2) a correlated double sampling stage (CDS) which is arranged under the assigned sensor (2) or in a region of the semiconductor chip (1) outside the region of the sensor array.

5. Arrangement according to any one of the previous claims, **characterized in that** the arrangement comprises means for setting a defined temperature over the sensor array.

6. Arrangement according to any one of the previous claims, **characterized in that** the arrangement comprises means for immobilizing living cells (4) over sensors (2) of the sensor array.

7. Arrangement according to claim 6, **characterized in that** as a means for immobilizing living cells (4) a filter membrane is arranged in fluid contact with the sensors (2) of the sensor array.

8. Arrangement according to claim 6, **characterized in that** the arrangement comprises at least one magnetic field-creating device, which is configured to create a magnetic field over the sensors (2) of the sensor array, and wherein living cells (4) are bound to magnetic particles (3).

9. Arrangement according to claim 8, **characterized in that** the living cells (4) are immobilized by the magnetic field over the sensor array in the form of an essentially uniformly thick layer of magnetic particles (3) with cells (4) embedded in the matrix of magnetic particles (3).

10. Arrangement according to claim 9, **characterized in that** the magnetic field is switchable between an on and an off state, in particular for the removal of dead or damaged cells (4) in the state of the switched-off magnetic field.

11. Arrangement according to any one of the previous claims, **characterized in that** the semiconductor chip (1) is surrounded by a flow cell and the sensors (2) of the sensor array are arranged in fluid contact with a flow channel of the flow cell.

12. Method for measuring cell vitalities with an arrangement according to any one of the previous claims, **characterized in that** the pH-value and/or the pO₂-value in the vicinity of a living cell (4) is measured by at least one sensor (2) of the sensor array.

13. Method according to claim 12, **characterized in that** over the sensor array a temperature optimal for cell vitality is set, in particular in the region of 37°C, and/or substances are supplied to the cells (4) which favor the cell vitality, in particular nutrient solution and/or oxygen, and/or substances are supplied to the cells (4) which impair the cell vitality, in particular antibiotics.

## Revendications

1. Agencement de mesure de vitalités de cellule, avec un réseau de capteurs, qui est réalisé sur une surface d'une puce à semi-conducteur (1), dans lequel la puce à semi-conducteur (1) comprend des circuits intégrés et un circuit intégré est associé à chaque capteur (2) du réseau de capteurs pour le traitement des signaux de mesure du capteur (2) respectif, dans lequel les circuits intégrés sont réalisés dans la puce à semi-conducteur (1) spatialement respectivement en dessous du capteur (2) associé et dans lequel des capteurs (2) adjacents du réseau de capteurs présentent une distance des points médians des capteurs (2) adjacents dans la plage des micromètres,
**caractérisé en ce**
**que** le circuit intégré d'un capteur (2) comprend deux transistors de commutation (M1, M2) pour la commutation du capteur (2) entre deux valeurs de tension, d'une première et d'une seconde tension, et
**que** le circuit intégré d'un capteur (2) comprend un transistor en tant que suiveur de tension (M3) et un transistor de sélection (M4) pour la sélection électrique ciblée du capteur (2) respectif selon des colonnes et des lignes du réseau de capteurs.

2. Agencement selon la revendication 1, **caractérisé en ce que** la distance des points médians de capteurs (2) adjacents se trouve dans l'ordre de grandeur des cellules (4) vivantes, en particulier dans la plage de 1 à 100 micromètres, de préférence dans la plage de 1 à 10 micromètres.

3. Agencement selon la revendication 1 ou 2, **caractérisé en ce que** les capteurs (2) sont des capteurs électrochimiques, en particulier des capteurs électrochimiques ampérométriques ou coulométriques, et/ou les capteurs (2) comprennent respectivement au moins une électrode interdigitale en tant qu'électrode de travail.

4. Agencement selon l'une quelconque des revendications précédentes, **caractérisé en ce que** le circuit intégré comprend pour la suppression de signaux de bruits électriques et pour la suppression de dérive électrique d'un capteur (2) pour chaque capteur (2) un niveau de double échantillonnage (CDS) corrélé qui est agencé en dessous du capteur (2) associé ou dans une zone de la puce à semi-conducteur (1) en dehors de la zone du réseau de capteurs.

5. Agencement selon l'une quelconque des revendications précédentes, **caractérisé en ce que** l'agencement comprend des moyens pour le réglage d'une température définie sur le réseau de capteurs.

6. Agencement selon l'une quelconque des revendications précédentes, **caractérisé en ce que** l'agencement comprend des moyens pour la fixation de cellules (4) vivantes sur des capteurs (2) du réseau de capteurs.

7. Agencement selon la revendication 6, **caractérisé en ce qu'**une membrane de filtre est agencée en contact fluidique avec les capteurs (2) du réseau de capteurs en tant que moyens de fixation de cellules (4) vivantes.

8. Agencement selon la revendication 6, **caractérisé en ce que** l'agencement comprend au moins un dispositif générant un champ magnétique qui est réalisé afin de générer un champ magnétique sur les capteurs (2) du réseau de capteurs, et dans lequel des cellules (4) vivantes sont liées à des particules magnétiques (3).

9. Agencement selon la revendication 8, **caractérisé en ce que** les cellules (4) vivantes sont fixées par le champ magnétique sur le réseau de capteurs, sous la forme d'une couche épaisse sensiblement uniformément de particules magnétiques (3) avec des cellules (4) intégrées dans la matrice de particules magnétiques (3).

10. Agencement selon la revendication 9, **caractérisé en ce que** le champ magnétique peut être commuté entre un état marche et un état arrêt, en particulier pour le retrait de cellules (4) mortes ou endommagées dans l'état du champ magnétique arrêté.

11. Agencement selon l'une quelconque des revendications précédentes, **caractérisé en ce que** la puce à semi-conducteur (1) est comprise par une cellule de débit et les capteurs (2) du réseau de capteurs sont agencés en contact fluidique avec un canal de débit de la cellule de débit.

12. Procédé de mesure de vitalités de cellule avec un agencement selon l'une quelconque des revendications précédentes, **caractérisé en ce que** la valeur pH et/ou la valeur pO₂ est mesurée par au moins un capteur (2) du réseau de capteurs dans l'environnement d'une cellule (4) vivante.

13. Procédé selon la revendication 12, **caractérisé en ce qu'**une température optimale pour la vitalité de cellule est réglée sur le réseau de capteurs, en particulier dans la plage de 37 °C, et/ou des substances sont amenées aux cellules (4) qui favorisent la vitalité de cellule, en particulier une solution nutritive et/ou de l'oxygène, et/ou des substances sont amenées aux cellules (4) qui dégradent la vitalité de cellule, en particulier des antibiotiques.
